# EUROPEAN PATENT APPLICATION

(11) **EP 3 202 780 A1**
(43) Date of publication of application: **09.08.2017**
(21) Application number: 16305140.2
(22) Date of filing: 05.02.2016
(51) Int. Cl.: C07K 14/135, C12N 15/62, C12N 15/63, C12N 5/10, A61K 39/155

(54) **FUSION PROTEIN COMPRISING THE N PROTEIN OF A VIRUS OF THE PNEUMOVIRINAE SUBFAMILY**

(71) Applicant: Institut National de la Recherche Agronomique (INRA), 75338 Paris Cedex 07 (FR)
(72) Inventor: ELEOUET, Jean-François, 91650 Breuillet (FR); RIFFAULT, Sabine, 78000 Versailles (FR); HERVE, Pierre-Louis, 78460 Chevreuse (FR)
(74) Representative: Lavoix

(57) **Abstract**

The invention relates to a fusion protein comprising a N protein from a virus of the *Pneumovirinae* subfamily, *Pneumovirinae* subfamily, useful in immunization and vaccination methods.

## Description

The invention relates to a fusion protein comprising a N protein from a virus of the *Pneumovirinae* subfamily, useful in immunization and vaccination methods.

Respiratory syncytial virus (RSV) is the major cause of acute respiratory infection in the pediatric population. Bronchiolitis and pneumonia, the most severe clinical manifestations of RSV, affect 25 to 40 % of infected infants and children. Furthermore, acute RSV infection in young infants leads to hospitalization in 0.5 to 2 % of cases, eventually resulting in death. In addition, elderly, immunocompromised adults and individuals with cardiopulmonary diseases are also susceptible to acute and fatal RSV infections.

Despite the high public health incidence of RSV infections and associated diseases, there is currently no available vaccine against this virus.

RSV belongs to the *Mononegavirales* order, to the *Paramyxoviridae* family and to the *Pneumovirinae* subfamily. The viral particles are surrounded by a lipid envelope containing two major proteins, the fusion protein (F) and the glycoprotein (G). Inside the particles there is a single-stranded RNA of negative polarity of approximately 15 kb, associated with the nucleocapsid protein (N). That RNA-N complex (or ribonucleoprotein complex) constitutes the matrix of the polymerase complex constituted by the L protein (large fragment), which is the RNA-dependent RNA polymerase, and its cofactor P (phosphoprotein), which are also present in the virions.

The inventors previously developed a method for obtaining the N protein of RSV in soluble recombinant form having a ring-like structure, and its use as a vaccine. The N protein is in the form of very regular rings containing 10 to 11 molecules of N protein and a RNA of approximately 70 bases. The N protein can be coexpressed in *E. coli* with the P protein of RSV, more particularly its C-terminal portion, fused to glutathione-S-transferase (GST), which allows the complexes to be purified by affinity with sepharose-glutathione beads. Those ring-like structures induce a strong immune response in the mouse, especially by intranasal administration, also in the absence of adjuvant. That method is described in the patent application FR 05 04 426.

The inventors have now shown that it is possible to use those ring-like structures as a vaccinal vector, even for conformational epitopes in which the conservation of the three-dimensional structure is essential to their function. The feasibility of that technology has been demonstrated by inserting an RSV conformational epitope in frame at the loop of the β hairpin of the RSV N protein, said epitope being the RSV F antigenic site II (Fsll) targeted by palivizumab. This fusion protein was coexpressed in *E. coli* with the C-terminal portion of the P protein (amino acids 161-241) fused to GST. Analysis of the purified structures using an electron microscope showed that the insertion of Fsll at the G106 residue of N protein (in the loop of the β hairpin), still allows the N protein to be produced in the form of rings, similarly to fusion proteins wherein Fsll is fused at the N-terminal end of the N protein. Indirect ELISA permitted to confirm the effective presence of the Fsll epitopes on N nanorings. Overall, these results indicate that the insertion of Fsll in the N protein at the loop of the β hairpin, in particular at the G106 position, allowed the formation of N-Fsll nanorings. Moreover, N-Fsll nanorings were efficiently recognized by anti-F antibodies. Furthermore, the inventors showed that insertion at the loop of the β hairpin, in particular at the G106 position, of the N protein led to a better preservation of the antigenic conformation of Fsll, since these fusion proteins were significantly better at inducing F-specific antibodies than fusion proteins wherein Fsll is fused at the N-terminal end of the N protein. Their results show that N nanorings is an efficient platform for the presentation of conformational epitopes requiring a specific set up to maintain their antigenicity.

Because the N-RNA rings are extremely immunogenic, the inventors injected those structures into mice in order to see if they could induce a strong antibody response, especially against RSV. The responses were compared with those obtained against the post fusion F protein and N protein. Post fusion F protein is a predominant F conformation found on the virion surface, which conserved the secondary structure of Fsll. The results obtained showed a similar response when the fusion protein (with insertion at G106) is used than when F and N proteins are used together. Furthermore, Fsll-fused chimeric nanorings are easy to produce and purify and thus represent a competitive vaccinal strategy over recombinant soluble F proteins the production of which is more challenging, since soluble F protein has to be produced in mammalian cells and is more difficult and more expensive to obtain than N nanorings carrying foreign epitopes. These results permit to confirm that the fusion proteins according to the invention are suitable vaccinal vectors.

The possibility of inserting a peptide of interest in the N protein and of purifying the complexes in the form of soluble rings on an industrial scale and at moderate cost (in particular in *E. coli*) thus permits the development of vaccines using any type of epitope (linear or conformational epitope) that is not very immunogenic or is not immunogenic when injected on its own or is difficult to produce on an industrial scale.

Furthermore the inventors have shown that those fusion protein or complexes are adsorbed and are effectively internalized by different cell types. Those results therefore highlight the value of such fusion protein, or complex, as an antigen vector for vaccination.

The invention thus relates to a fusion protein comprising or consisting of a N protein from a virus of the *Pneumovirinae* subfamily and a peptide of interest, said peptide of interest being inserted in frame at the loop of the β hairpin of the N-terminal domain of the N protein.

### Detailed description of the invention

### Pneumovirinae

The *Pneumovirinae* subfamily encompasses two genera, the *Pneumovirus* and the *Metapneumovirus,* the latter including the human *Metapneumovirus.* Human respiratory syncytial virus (RSV) constitutes the prototype virus of the genus *Pneumovirus.* The *Pneumovirus* subfamily also includes the bovine and murine strains of RSV. According to the invention said virus of the *Pneumovirinae* subfamily may be selected from the group consisting of the human *Metapneumovirus,* human and bovine respiratory syncytial virus (RSV). Preferably, said virus of the *Pneumovirinae* subfamily is selected from the group consisting of human or bovine RSV. Most preferably, said virus of the *Pneumovirinae* subfamily is human RSV.

Unless specified otherwise, "respiratory syncytial virus" is generally understood as meaning RSV, whatever its form (human, bovine, etc.), the subgroup (for example subgroups A, B and S identified in human RSV) or the strain in question.

### Fusion proteins

The invention relates to a fusion protein comprising or consisting of a N protein from a virus of the *Pneumovirinae* subfamily and a peptide of interest, said peptide of interest being inserted in frame at the loop of the β hairpin of the N-terminal domain of the N protein.

"N protein" denotes the nucleocapsid protein of the virus of the *Pneumovirinae* subfamily, which forms helical structures to surround the viral genome. The N protein of human RSV, in particular of human RSV Long strain, typically consists of a sequence of 391 amino acids and is typically of sequence SEQ ID NO: 1. The N protein of bovine RSV likewise typically consists of 391 amino acids, and is typically of sequence SEQ ID NO: 2. The N protein of the human *Metapneumovirus,* in particular of the human *Metapneumovirus* strain 00-1, is typically described under the Swissprot database accession number Q91 F57 (as of 18 April 2006), and is typically of sequence SEQ ID NO: 3.

Each N protein comprises a core region comprising two domains named N and C-terminal domains (abbreviated NTD and CTD respectively) that are connected through a hinge region. The NTD of human RSV N protein typically consists in 218 residues (typically from amino acid 36 to 253 of SEQ ID NO: 1) and comprises a long β hairpin.

The expression "N protein" may denote a whole N protein or a truncated N protein. According to the invention, the N protein may be a native N protein, that is to say a N protein naturally present in a *Pneumovirinae* virus, or may have been modified in the region defined by the last 25, preferably the last 22, 20, 18, 15, 14, 12, 10, 8, 6 or 5, C-terminal amino acids, provided that the modified N protein retains the ability to interact with the P protein. Such modifications typically consist in the deletion, substitution and/or insertion of one or more amino acids (for example from 1 to 25, from 5 to 25 from 1 to 22, from 1 to 20, from 1 to 18, from 1 to 15, from 1 to 14, from 1 to 12, from 1 to 10, or from 1 to 8, from 1 to 6, or alternatively from 1 to 5 contiguous or non-contiguous amino acids) in the C-terminal sequence of the N protein. Preferably such modifications consist in the deletion of one or more amino acids (for example from 1 to 25, from 5 to 25 from 1 to 22, from 1 to 20, from 1 to 18, from 1 to 15, from 1 to 14, from 1 to 12, from 1 to 11, from 1 to 10, from 1 to 9, from 1 to 8, from 1 to 7, from 1 to 6, or from 1 to 5, from 1 to 4, from 1 to 3, from 1 to 2 contiguous or non-contiguous amino acids or alternatively a deletion of the last amino acid) in the C-terminal sequence of the N protein, thus forming a truncated N protein. An example of a sequence modification of the N protein can consist in a deletion of the 6 or 12 C-terminal amino acids, these truncated N proteins (NΔ6C and NΔ12C) still being capable of interacting with the P protein. According to the invention, the N protein may keep its capacity of interacting with the P protein and be further modified. The person skilled in the art knows, or is capable of determining modified or truncated N proteins, which are capable of interacting with the P protein (see for example Galloux et al. 2012, Journal of Virology; 86: 8375-8387).

The expression "loop of the β hairpin of the N-terminal domain of the N protein" refers to the region corresponding to the region between residues 103-109 of human RSV protein N, in particular of SEQ ID NO: 1. Preferably, the loop of the β hairpin of the N-terminal domain of the N protein, in particular of the RSV N protein, more particularly of the human RSV N protein, consists of the sequence SEQ ID NO: 4. In a preferred embodiment, the peptide of interest is inserted between amino acids corresponding to amino acids 1104/N105, 1104/G106, 1104/K107, N105/K107 or G106/K107 of human RSV N protein, in particular of SEQ ID NO: 1. Preferably the peptide of interest is inserted between amino acids corresponding to amino acids G106/K107 of human RSV N protein, in particular of SEQ ID NO: 1. This construction allows the fusion protein N protein-peptide of interest to be incorporated into ring-like structures comprising ten or eleven proteins N.

A "peptide of interest" encompasses any peptide, such as, for example, a marker peptide, or a peptide of therapeutic or vaccinal interest.

Preferably the peptide of interest is constituted of at most 250 amino acids or at most 200 amino acids or at most 100 amino acids, or at most 50 amino acids, at most 45 amino acids, at most 40 amino acids, at most 38 amino acids at most 30 amino acids or at most 25 amino acids, or at most 20 amino acids. It can include 2 or 4 cysteines located on either side of the epitope to stabilize it.

A peptide of therapeutic interest may be, for example, an antiangiogenic peptide, for example RGD or a sequence including RGD, endostatin, or a proapoptotic peptide such as fragments from the apoptosis-inducing factor (AlF), which can be used as anticancer agents, a peptide capable of interacting specifically with viral proteins and interfering with the mechanisms that permit the replication of a virus, or a toxin.

The peptide of interest may be an antigen, in particular an antigenic protein of vaccinal interest. "Antigen" or "Ag" is understood as being a sequence of peptidic or glycopeptidic nature that is capable of inducing an immune response in a host to which it is administered. Accordingly, an antigen may be a small peptide potentially corresponding to an epitope.

An "epitope" is the part of an antigen that is recognized by an antibody or by a lymphocyte receptor. The term "epitope" encompasses linear epitopes and conformational epitopes. A linear epitope is an epitope that is recognized by antibodies by its linear sequence of amino acids, i.e. its primary structure. In contrast, a conformational epitope is recognized by antibodies by its specific three-dimensional shape and its protein structure. An antigen within the scope of the invention may be constituted by an epitope, may comprise an epitope or may be an antigenic peptide. Preferably an antigen within the scope of the invention is an epitope, and most preferably an antigen within the scope of the invention is a conformational epitope.

Preferably, the antigen is an antigen derived from a pathogenic microorganism, such as a virus, a bacterium, a fungus or a parasitic metazoan or protozoan organism.

Examples of viruses include human immunodeficiency virus (HIV), hepatitis B virus (HBV), hepatitis C virus (HCV), herpes virus (*Herpes simplex),* flu virus (influenza or avian flu virus), occidental Nile virus, yellow fever virus, cytomegalovirus, papillomavirus (HPV), Epstein-Barr virus (EBV), RSV, Dengue virus and Chikungunya virus.

More preferably, the antigen is an antigen derived from a virus of the *Pneumovirinae* subfamily, most preferably from RSV, in particular from human RSV.

According to the invention, when the peptide of interest is from a virus of the *Pneumovirinae* subfamily, it preferably derives from the same virus as the N protein of the fusion protein.

According to the invention, the antigen may be a viral glycoprotein such as the RSV fusion protein (F), influenza virus hemagglutinin, or HIV gp120 protein, a bacterial anatoxin such as the tetanus anatoxin, or an epitope thereof.

In one embodiment of the invention the antigen is an epitope derived from the F protein from a virus of the *Pneumovirinae* subfamily, preferably from RSV, most preferably from human RSV. In a preferred embodiment the antigen is the epitope Fsll.

The "epitope Fsll" is a well-characterized conformational neutralizing epitope targeted by the humanized monoclonal antibody named palivizumab. It corresponds to the antigenic site II located on the F protein of human RSV. Fsll (corresponding to amino acids 255 to 275 of F protein of human RSV) typically consists of the sequence SEQ ID NO: 5. It preferably folds as a helix-loop-helix secondary structure that is essential for Palivizumab binding. This structure is conserved in the two predominant F conformations found on the virion surface, i.e. the metastable prefusion state (responsible for the initialization of the fusion between viral and cellular membranes) and the highly stable postfusion state. Antibodies directed against Fsll such as Palivizumab neutralize infection by blocking the fusion between viral and cellular membranes, thus aborting virus entry. The Fsll epitope according to the invention may comprise or consist of a continuous span of amino acids comprising or consisting of amino acids corresponding to amino acids 255 to 275 of F protein of human RSV. In a preferred embodiment Fsll epitope according to the invention comprises or consists of a continuous span of at least 22, 23, 24, 25, 26, 27, 28, 29, 30, 31, 32, 33, 34, 35, 36, 37, 38, 39, 40, 41, 42, 43, 44, 45, 46, 47, 48, 49, 50 amino acids. Preferably the Fsll epitope according to the invention comprises or consists of a continuous span of amino acids comprising or consisting of amino acids corresponding to amino acids 248 to 285 of F protein of human RSV of sequence SEQ ID NO: 6. Most preferably, the Fsll epitope according to the invention consists of a continuous span of amino acids corresponding to amino acids 248 to 285 of F protein of human RSV of sequence SEQ ID NO: 6.

In some embodiments, the fusion protein according to the invention further comprises supplementary peptide(s) of interest. Said supplementary peptide of interest may be the same as the first peptide of interest (in other words, the fusion peptide comprises several copies of the same peptide of interest) or a different peptide. Said supplementary peptide of interest can be inserted in frame at one end of the first peptide of interest or inserted elsewhere in the sequence of the N protein, for example at the N-terminal end or at the C-terminal end of the N protein or at a different site in the loop of the β hairpin of the N-terminal domain of the N protein. For example the first peptide of interest may be an epitope which can be fused to a supplementary peptide of interest, in particular another epitope of the same origin.

The fusion protein may also be a chimeric protein comprising a "linker sequence" fused at one or two ends of the peptide of interest. The linker sequence is a polypeptide which typically comprises or consists of up to 30 amino acids, preferably up to 20 amino acids, more preferably up to 10 amino acids, more preferably up to 5 amino acids and which acts as a spacer between the N protein and the peptide of interest, which enables each of those proteins to be correctly folded.

### Complexes

The invention also relates to a complex, also named fusion protein /P protein complex, comprising or consisting of a fusion protein according to the invention and a P protein, wherein the N and the P protein are from the same virus of the *Pneumovirinae* subfamily.

The expression "P protein" denotes the phosphoprotein forming part of the polymerase complex of a virus of the *Pneumovirinae* subfamily. The P protein is a cofactor of the viral polymerase (replicase/transcriptase) and can be phosphorylated. The sequences of the P protein of *Pneumovirinae* subfamily are known to the person skilled in the art. For example, the P protein of human RSV Long strain has a sequence of 241 amino acids, which is typically shown in SEQ ID NO: 7. The P protein of bovine RSV also consists of 241 amino acids and is typically of sequence SEQ ID NO: 8. The P protein of the human *Metapneumovirus* strain 00-1 is described under the Swissprot database accession number Q91 KZ5 and is typically of sequence SEQ ID NO: 9.

The expression "P protein" may denote a whole P protein, a truncated P protein or a fragment of the P protein.

The expression "truncated P protein" denotes a P protein in which one or more contiguous amino acid sequences have been deleted. It may be the truncation of a C-terminal sequence, a N-terminal sequence, a sequence that is "internal" relative to the primary structure of the P protein, or a combination of these truncations. Preferably the P protein is a truncated protein comprising C-terminal fragment of the P protein.

A "C-terminal fragment of the P protein" or "PΔN" is understood as meaning a P protein in which one or more consecutive amino acids have been deleted starting from the N-terminal end. The C-terminal fragment of the RSV P protein may be selected, for example, from the group constituted by PΔ120N (amino acids 120 to 241 of P), PΔ150N (amino acids 150 to 241 of P), PΔ161N (amino acids 161 to 241 of P), PΔ180N (amino acids 180 to 241 of P), PΔ200N (amino acids 200 to 241 of P), PΔ220N (amino acids 220 to 241 of P), PΔ230N (amino acids 230 to 241 of P) and PΔ233N (amino acids 233 to 241 of P). Preferably, a C-terminal fragment of the P protein denotes a chain of amino acids situated in the C-terminal half of the primary structure of the P protein (when the number of amino acids in the sequence is odd, an additional amino acid can be allocated arbitrarily to the C-terminal half of the protein relative to the N-terminal half). For example, in the case of the RSV P protein, which comprises 241 amino acids, PΔ161N denotes a C-terminal fragment constituted by amino acids 161 to 241 of the P protein.

The P protein of the *Pneumovirinae* forms homo-oligomers, in particular homotetramers, for example in the RSV. In the case of human RSV, a domain of the P protein capable of oligomerization (P-P oligomerization) has been mapped in the region of amino acids 120 to 150 of that protein (Castagné et al., 2004; Journal of General Virology; 85: 1643-1653). Thus, for example, the fragment PΔ161N does not form oligomers. The inventors have demonstrated that this fragment promotes the preparation of large quantities of N protein as compared with whole P protein. The smaller deletion mutants of PΔ161N, down to PΔ233N (amino acids 233 to 241), which contains only 9 amino acids, are capable of interacting with the N protein, and are no longer capable of oligomerization as they no longer contain the oligomerization domain P, but permit to achieve yields comparable with those of PΔ161N.

The use of C-terminal fragments of the P protein which contain the interaction domain with the N protein therefore permitting the interaction of the protein P fragments with the N protein, but in which the oligomerization domain P has been deleted, permits the formation of complexes and the production of those complexes with a high yield. Without wishing to be limited to a particular mechanism, it is assumed that the absence of the oligomerization domain P avoids problems of insolubility of the complexes associated with interactions between P proteins of those complexes.

Thus according to the invention, said P protein is preferably a truncated P protein which does not contain the oligomerization domain P and which comprises a binding domain to the N protein. Since the domain of interaction of the *Pneumovirinae* P protein with the N protein has been mapped in the region of the C-terminal end, examples of truncated P protein preferably include a C-terminal fragment of the P protein. Preferably a truncated P protein comprising a C-terminal fragment of the RSV P protein according to the invention comprises the last 9 C-terminal amino acids of the RSV P protein and is devoid of at least the 119, preferably the 149, more preferably the 160 N-terminal amino acids of the RSV P protein.

The person skilled in the art knows, or is capable of determining, truncated P proteins, or more specifically C-terminal fragments of the P protein, which are capable of interacting with the fusion protein and which does not contain the oligomerization domain P. For example, the strategy of coexpressing the N and P proteins in *E. coli* described by Castagné et al. (2004, Journal of General Virology; 85: 1643-1653) may be used to map the interaction domain between P and N.

The sequences of the P and N proteins described above are illustrative in nature, it being possible for the sequences to exhibit variations according to the particular strain under consideration for a given virus. Accordingly, the amino acid positions mentioned in the application are indicated relative to those reference sequences. The person skilled in the art is wholly capable of identifying the corresponding domains in virus strains other than those exemplified, especially with the aid of sequence alignments carried out, for example, using softwares such as Clustalw.

The coding sequences of these N and P proteins of viruses of the *Pneumovirinae* subfamily are also known to the person skilled in the art.

Advantageously, a protein label may be fused at the N protein or the P protein in order to facilitate the purification or the detection of the fusion proteins or complexes.

Advantageously, the P protein, for example the truncated P protein, in particular the C-terminal fragment of the P protein, is expressed in the form of a fusion protein with a protein that facilitates the purification of the complexes, especially a protein which can be used in affinity chromatography. It may be a protein tag, such as glutathione-S-transferase (GST), in which case the P protein-GST fusion protein can be isolated by chromatography on a solid support coupled with glutathione. Other labels or "tag", such as polyhistidine or "His-Tag", may be used.

The term "protein label", also called "protein tag", denotes a protein which is used fused to a protein in order to facilitate the purification or the detection thereof. Protein tags are known to the person skilled in the art. Examples of protein tags include glutathione-S-transferase (GST) or the histidine tags, which are sequences generally comprising a chain of from 4 to 10 histidine residues. The protein tag can later be removed by enzymatic cleavage. For example, GST may be removed by cleavage with thrombin or with any other suitable enzyme.

### Nucleic acids, expression vectors and host cells

The invention relates also to the nucleic acids coding for the above-mentioned fusion proteins.

A "coding sequence" denotes a nucleotide sequence which, when expressed, results in the production of a RNA, of a polypeptide, of a protein, etc. A coding sequence of a protein generally contains a start codon (ATG) and a stop codon.

"Express" or "expression" means to permit or enable the information contained in a gene or a DNA sequence to manifest itself, for example by producing a protein by activation of the cell functions involved in the transcription and translation of the genetic sequence or of corresponding DNA. The term "coexpression" is used when the information contained in two genes or DNA sequences is expressed in the same host cell.

Any conventional molecular biological, microbiological or recombinant DNA technique may be used to produce the fusion proteins or the complexes according to the invention. Such techniques are within the scope of the person skilled in the art and have been described, especially in Sambrook, Fritsch & Maniatis, Molecular Cloning: A Laboratory Manual, Second Edition (1989) Cold Spring Harbor Laboratory Press, Cold Spring Harbor, New York ("Sambrook *et al*., 1989"); DNA Cloning: A Practical Approach, Volumes I and II (D.N. Glover ed. 1985); Oligonucleotide Synthesis (M.J. Gait ed. 1984); Nucleic Acid Hybridization [B.D. Hames & S.J. Higgins eds. (1985)]; Transcription and Translation [B.D. Hames & S.J. Higgins, eds. (1984)]; Animal Cell Culture [R.I. Freshney, ed. (1986)]; Immobilized Cells and Enzymes [IRL Press, (1986)]; B. Perbal, A Practical Guide To Molecular Cloning (1984); F.M. Ausubel *et al*. (eds.), Current Protocols in Molecular Biology, John Wiley & Sons, Inc. (1994).

The invention therefore relates to nucleic acids (cDNA, genomic DNA, synthetic DNA, or RNA) encoding the fusion proteins according to the invention. The nucleic acid may be double-stranded or single-stranded (that is to say a sense or antisense strand). The nucleic acids are not limited to the sequence encoding the fusion protein and may include coding or non-coding sequences upstream or downstream of the sequence encoding the fusion protein.

The invention also relates to an expression vector comprising a nucleic acid coding for the fusion protein according to the invention. Said expression vector may further comprise a nucleic acid coding for a P protein from the same virus of the *Pneumovirinae* subfamily as the N protein of the fusion protein. Such vectors may contain a transcription-regulating element functionally linked with the DNA.

The invention also relates to an expression vector comprising a nucleic acid coding for the fusion protein according to the invention and preferably further comprising a nucleic acid coding for a P protein from the same virus of the *Pneumovirinae* subfamily as the N protein of the fusion protein.

A coding sequence is "functionally linked with" transcription sequences when a RNA polymerase transcribes the coding sequence into RNA, in particular into mRNA, which may subsequently be spliced, if it contains introns, and translated into the protein encoded by the coding sequence.

The expressions "vector" and "expression vector" denote the vehicle by which a DNA or RNA sequence (for example a heterologous gene) can be introduced into a host cell in order to transform the host cell and promote the expression of the sequence that has been introduced. Examples of vectors include plasmids, phages, and viruses. The most common vectors are plasmids, which are autonomous replication units, generally of bacterial origin, and which can be in the form of double-stranded DNA. The plasmids can readily integrate an exogenous DNA sequence, which can then readily be introduced into a suitable host. A plasmid vector generally contains a coding DNA sequence, a promoter DNA sequence and has one or more restriction sites permitting the introduction of exogenous DNA. Non-limiting examples of plasmids include the plasmids pKK (Clonetech), pUC and pET (Novagen, Inc., Madison, WI), pRSET or pREP (Invitrogen, San Diego, CA), pMAL (New England Biolabs, Beverly, MA), or pGEX-4T-3 (Pharmacia).

The invention also relates to a pair of expression vectors comprising a first expression vector comprising a nucleic acid coding for a fusion protein according to the invention and a second expression vector comprising a nucleic acid coding for a P protein from the same virus of the *Pneumovirinae* subfamily as the N protein of the fusion protein encoded by the first expression vector.

Specific examples of the vectors according to the invention are described in the examples below.
The invention also relates to a host cell containing:
(i) an expression vector according to the invention or
(ii) a pair of expression vectors according to the invention.

These host cells are "transformed" with said vectors.
The invention also relates to a host cell containing:
(i) an expression vector according to the invention or
(ii) a pair of expression vectors comprising a first expression vector as defined previously and a second expression vector comprising a nucleic acid coding for a P protein from the same virus of the *Pneumovirinae* subfamily as the N protein of the fusion protein encoded by the first expression vector.

"Host cell" is understood as meaning any cell or organism, in particular unicellular organism, which is selected, modified, cultivated or engineered for the production of a substance by the cell, for example the expression by the cell of a gene, a DNA or RNA sequence, a protein or an enzyme.

An "expression system" denotes a host cell and a compatible vector, which are used under suitable conditions to produce a protein encoded by an exogenous DNA carried by the vector and introduced into the host cell. Conventional expression systems include *E. coli* host cells and plasmid vectors, insect cells and Baculovirus vectors, or mammalian cells and vectors with strong promoters of viral origin (for example cytomegalovirus).

The expression system according to the invention is advantageously a bacterial expression system, in particular in *E. coli*, with, for example, pGEX-4T-3 as vector. Bacterial systems are indeed the expression systems that generally allow the highest production yields to be obtained.

### Methods for producing fusion protein and complex

The invention also relates to a method for producing a fusion protein according to the invention, said method comprising the culture of a host cell according to the invention under conditions permitting the expression of the fusion protein, and optionally the purification of the expressed fusion protein.

The inventors have previously shown that the N protein of a virus of the *Pneumovirinae* subfamily can be produced in a coexpression system with the P protein of the same virus of the *Pneumovirinae* subfamily. The same system can be used to express the fusion protein of the invention in the form of a complex with the P protein, and then optionally purify the fusion protein from that complex.

The invention relates also to a method for producing a fusion protein according to the invention or a complex according to the invention, which method comprises optionally the transformation of a host cell and then culturing the host cell transformed with a vector comprising a nucleic acid coding for the fusion protein according to the invention and/or a pair of vectors according to the invention under conditions permitting their expression, and optionally the purification of the expressed fusion proteins or complexes. The culture conditions depend on the selected expression system (that is to say host cell and vector) and the determination of those conditions is within the scope of the person skilled in the art.

The invention further relates to a method for preparing complexes according to the invention, said method comprising the steps consisting in:
a) coexpressing a fusion protein according to the invention, with a P protein from the same virus of the *Pneumovirinae* subfamily as the N protein of the fusion protein;
b) collecting the so formed fusion protein /P protein complexes.

The invention also relates to a fusion protein /P protein complex, obtainable by a preparation method as described above.

Starting from those complexes, the fusion protein according to the invention can readily be isolated in the form of rings, with their RNA, for example by size exclusion chromatography (gel filtration). This separation may be carried out, where appropriate, after separation, by enzymatic cleavage of the P protein and the protein tag to which the P protein is optionally fused.

The invention therefore relates further to a method for preparing a fusion protein according to the invention, said method comprising the separation of the fusion protein starting from the fusion protein /P protein complex according to the invention.

The invention also relates to fusion proteins obtainable by the above method.

### Immunogenic, vaccinal or therapeutic compositions

The inventors have previously shown that the RSV N protein having a ring-like structure, is highly immunogenic and especially permits the stimulation of a local response, for example in the respiratory mucosa.

The invention therefore relates to the fusion protein and/or the complex according to the invention, for use as a vaccine. The invention also relates to the use of the fusion protein or the complex according to the invention for the manufacture of a vaccine. The invention further relates to a method of vaccination comprising the step of administering a prophylactically effective amount of a fusion protein according to the invention or a complex according to the invention in a subject in need thereof.

A "vaccine" denotes a biological preparation that provides active acquired immunity to a particular disease. According to the invention, a vaccine can prevent infection from various pathogens; in particular a vaccine according to the invention can be a vaccine against a *Pneumovirinae* infection, preferably against an RSV infection, most preferably against human RSV infection. Still preferably the vaccine according to the invention can prevent bronchiolitis or pneumonia.

As RSV is the leading cause of bronchiolitis and pneumonia in infants and young children, the invention relates to a fusion protein or a complex according to the invention for its use as described above, wherein the peptide of interest of the fusion protein is an epitope derived from the RSV F protein, for use for preventing bronchiolitis or pneumonia. The invention also relates to the use of the fusion protein or the complex according to the invention for the manufacture of a vaccine intended for preventing bronchiolitis or pneumonia. The invention further relates to a method of prevention of bronchiolitis or pneumonia in a subject, comprising administering a prophylactically effective amount of a fusion protein according to the invention or of a complex according to the invention wherein the peptide of interest of the fusion protein is an epitope derived from the RSV F protein.

The invention relates also to a method, in particular an in vitro method, for delivering a peptide into a cell, which method comprises delivering to said cell said peptide through the delivery of a fusion protein according to the invention in which the peptide of interest is the peptide to deliver.

The invention also proposes a pharmaceutical composition comprising a fusion protein and/or a complex according to the invention, and a pharmaceutically acceptable carrier.

According to an embodiment, the peptide of interest is an antigen and the pharmaceutical composition is an immunogenic or vaccinal composition. The inventors have demonstrated that the fusion protein according to the invention penetrates into the cells and that the N protein therefore constitutes a potential vector for peptides.

According to an embodiment, the pharmaceutical composition is suitable for immunogenic or vaccinal use.

The fusion protein or the complex according to the invention can be used for vaccination without any adverse effect. Consequently, an immunogenic or vaccinal composition according to the invention may comprise a fusion protein and/or a complex according to the invention, in association with a pharmaceutically acceptable carrier.

"Pharmaceutically acceptable carrier" is understood as meaning any solvent, dispersion medium, absorption-retarding agent etc. that does not produce any side effect, for example an allergic reaction, in humans or animals.

Examples of physiologically acceptable carriers are known to the person skilled in the art. Examples of liquid carriers include sterile aqueous solutions which do not contain any material other than the active ingredients and water, or which contain a buffer such as sodium phosphate at physiological pH value, with a physiological salinity, or both, such as a phosphate-buffered saline solution (PBS). Aqueous carriers can contain more than one buffer salt as well as salts such as sodium or potassium chloride, dextrose, polyethylene glycol and other solutes.

The compositions are administered in a manner compatible with the galenical formulation and in a therapeutically effective amount. The amount to be administered depends on the subject to be treated, on the ability of the subject's system to use the active ingredient, and on the desired degree of therapeutic effect. The precise amounts of fusion protein required for the administration depend on the choice of the physician and on the particularities of each individual.

Advantageously, when the pharmaceutical composition is an immunogenic or vaccinal composition, it may further comprise an adjuvant. An "adjuvant" denotes a product which increases, stimulates, activates, improves or modulates the immune reaction at cell or humoral level directed against an antigen administered simultaneously. Examples of conventional adjuvants include adjuvants containing bacterial antigens, such as Freund's complete adjuvant, LPS and its derivatives, bacterial toxins (cholera toxin and enterotoxin) and their detoxified mutants (for example LT(R192G)), oligonucleotide sequences containing CpG motifs, mineral adjuvants such as aluminium hydroxide (alum), calcium phosphate or potassium phosphate, oily emulsions and emulsifying agents (saponins, for example QS21), cytokines.

The immunogenic compositions according to the invention allow an immune response to be induced against the antigen in the vaccinated subject, or more specifically against the pathogen from which the antigen is derived.

The vaccinal compositions according to the invention allow protection to be conferred against an infection by a pathogen comprising the antigen, that is to say a reduction in the severity of the effects of such an infection as compared with a subject which has not been immunized with the vaccinal composition.

The invention relates also to the use of a vaccinal composition as defined above in a method of vaccination against the pathogen from which the antigen is derived.

The invention therefore relates to a vaccination method comprising at least one administration of a vaccinal composition according to the invention to a subject. Preferably, the vaccination method comprises a first administration to a subject of a vaccinal composition and at least one booster administration of said vaccinal composition to the same subject. The booster administrations, by reexposing the patient to the antigen, induce a stronger secondary immune response.

The vaccinal composition is advantageously administered in an amount that is effective in inducing a protective or therapeutic immune response to an infection by the pathogen from which the antigen is derived. The dosage naturally depends on the active ingredient in question, on the mode of administration, and on the age and condition of the subject. The amount of fusion protein or complex according to the invention per dose can be from 0.1 to 200 µg, preferably from 10 to 100 µg, per vaccinal dose.

The immunogenic, vaccinal, or pharmaceutical composition can be administered by any route, in particular by the mucosal (for example ocular, intranasal, oral) route or by the parenteral (for example subcutaneous, intradermal, intramuscular, intravenous or intraperitoneal) route.

The expression "subject" denotes a human being or a non-human animal, for example a bird or a mammal such as a bovine, an ovine, a rodent, a canine, in particular a dog, a feline, in particular a cat, a pig, a monkey, which has been exposed or is likely to be exposed to an infection by a *Pneumovirinae* virus or by any other pathology. A subject within the scope of the invention is preferably a human being or a bovine. A subject within the scope of the invention is preferably a human being, more preferably a child or an infant.

### Diagnostic applications

The insertion of an antigen comprising at least one epitope in the N protein of a *Pneumovirinae* virus additionally constitutes a reagent likely to be used in diagnostic applications for detecting antibodies directed against at least one of said epitopes carried by the fusion protein.

The invention therefore relates further to a diagnostic reagent comprising a fusion protein or a complex according to the invention wherein the peptide of interest of the fusion protein is an antigen.

A diagnostic kit comprising said reagent and appropriate detection means are likewise within the scope of the invention.

The invention also proposes the use of a fusion protein or a complex according to the invention, wherein the peptide of interest of the fusion protein is an antigen, in the *in vitro* or *in vivo* detection of antibodies directed against said antigen.

When the antigen comprises a single epitope, the detected antibodies are then specific for that epitope.

When the antigen comprises two or more than two different epitopes, the detected antibodies can be specific for one, two or more than two of the epitopes of said antigen.

The invention also relates to a method for detecting, in a biological sample, antibodies specific for an antigen, which method comprises the steps consisting in:
a) contacting said biological sample with a fusion protein according to the invention or a complex according to the invention wherein the peptide of interest of the fusion protein is an antigen,
b) detecting the resulting fusion protein/antibody complexes, or the resulting (fusion protein/P protein complex)/antibody complexes,
the presence of such complexes being indicative of the presence in the biological sample of antibodies specific for the antigen.

The biological sample may be a tissue sample obtained, for example, by muscular, hepatic, cardiac, cerebral, etc. biopsy, or a liquid sample, for example a biological liquid such as blood, plasma or cerebrospinal fluid.

The detection of the fusion protein/antibody complexes or the (fusion protein/P protein complex)/antibody complexes may be carried out by conventional means well known to the person skilled in the art, such as chromatography (size exclusion, affinity, etc.) or electrophoresis under non-denaturing conditions.

The detection of the fusion protein/antibody complexes or the (fusion protein/P protein complex)/antibody complexes may additionally be facilitated by labelling the N proteins in a detectable manner.

The following examples and figures illustrate the invention without limiting the scope thereof.

### Brief description of the figures

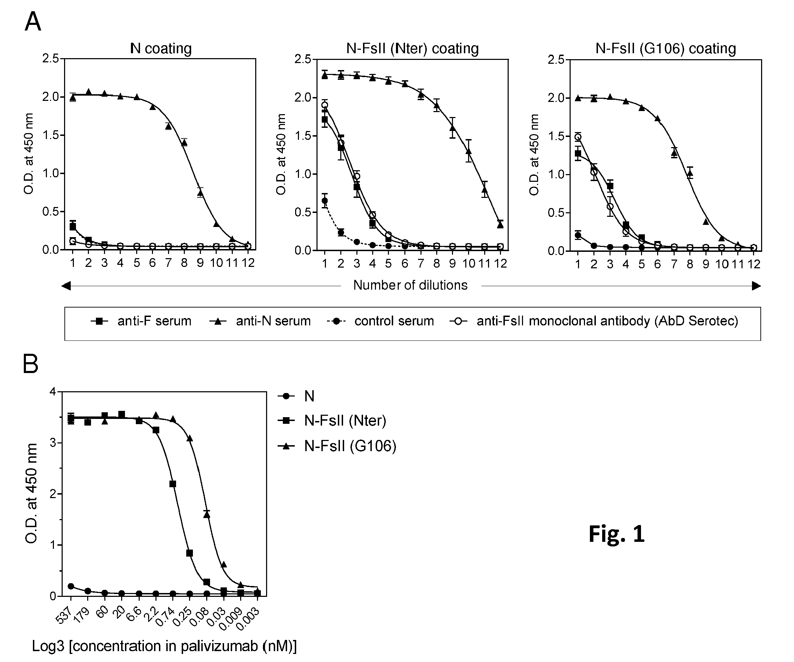

Figure 1: (A) Reactivity of N-Fsll chimeric nanorings with mice sera or Fsll-specific monoclonal antibody. Purified N-Fsll (Nter) (middle panel) and N-Fsll (G106) (right panel), or Fsll-free N nanorings (N, left panel) were coated on microplates. The reactivity of recombinant proteins with different antibodies was evaluated by applying 3 fold dilutions of pooled sera collected from postfusion F (F) immunized mice (anti-F serum, black squares), N nanorings immunized mice (anti-N serum, black triangles) or naive mice (control serum, black circles, or Fsll-specific monoclonal antibody (AbD Serotec [clone RSV3216]) (white squares). The first dilution was 1:30 for all samples. Binding of the antibodies was detected using anti-mice HRP-conjugated secondary antibodies and TMB colorimetric substrate, and optical density was measured at 450 nm. Of note, background was slightly higher when using N-Fsll (Nter) as a coating (middle panel). Data are mean ± SEM of triplicate to septuplicate values obtained from two independent experiments. Non-linear regression was done using Boltzmann sigmoidal equation. (B) Reactivity of N-Fsll chimeric nanorings with palivizumab. Purified N-Fsll (Nter) (squares), N-Fsll (G106) (triangles) or N (circles) were coated on microplates. Serial dilutions of palivizumab were then applied. Binding of palivizumab was detected using anti-human HRP-conjugated secondary antibody and TMB colorimetric substrate. Data are mean ± SEM of quadruplicate values. Non-linear regression was done using Boltzmann sigmoidal equation. Dissociation constants (Kd) were calculated from saturation curves obtained after baseline correction (N curve) and non-linear regression using Graph Pad prism software.

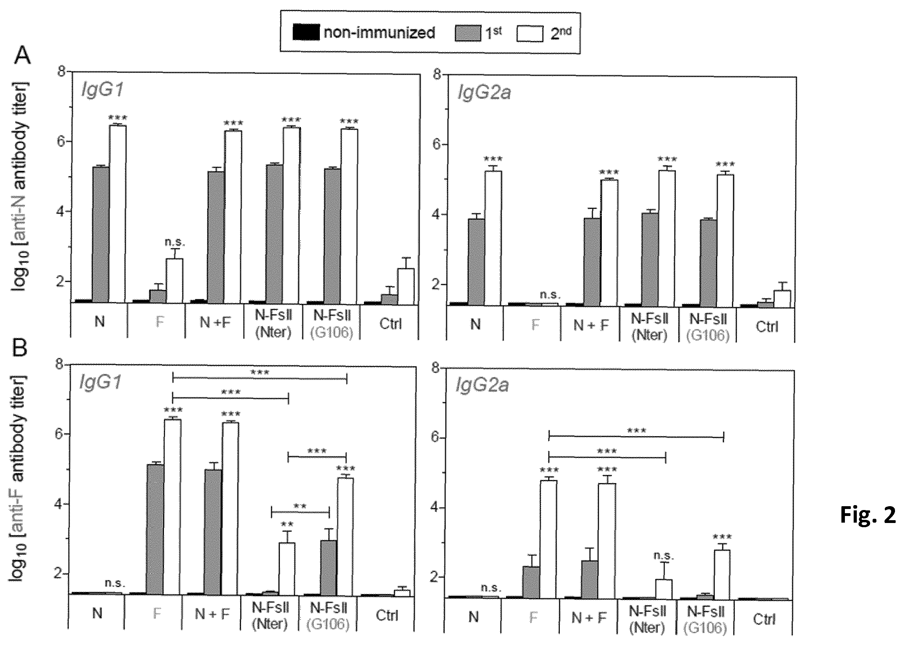

Figure 2: Measure of seric antibody responses in mice. BALB/c mice were immunized intranasally twice at two weeks interval with 2 µg of Fsll-free N nanorings (N) or postfusion F (F), 2 µg of N associated to 2 µg of F (N + F) or 2 µg of N-Fsll (Nter or G106) in NaCl 0.9 %. All immunogens were adjuvanted with 5 % MontanideTM Gel. As a control, mice received adjuvant intranasally in NaCl 0.9 % (Ctrl). Sera of vaccinated mice were collected before (non-immunized) and two weeks after the prime immunization (1st) or two weeks after the boost immunization (2nd). N (A) and F (B) specific IgG1 and IgG2a titers were determined individually in sera by indirect ELISA using N nanorings or F as the capture antigen, respectively. Data are mean + SEM of individual antibody titers for each group (n = 5-6 per experimental group). P values were determined according to the Student's T test (**, P<0.01; ***, P<0.001; n.s., non-significant). The level of significance measured between vaccinated groups was indicated above horizontal capped lines. The level of significance measured between each vaccinated groups and the negative control (Ctrl) group after the boost immunization was indicated above each histogram bar.

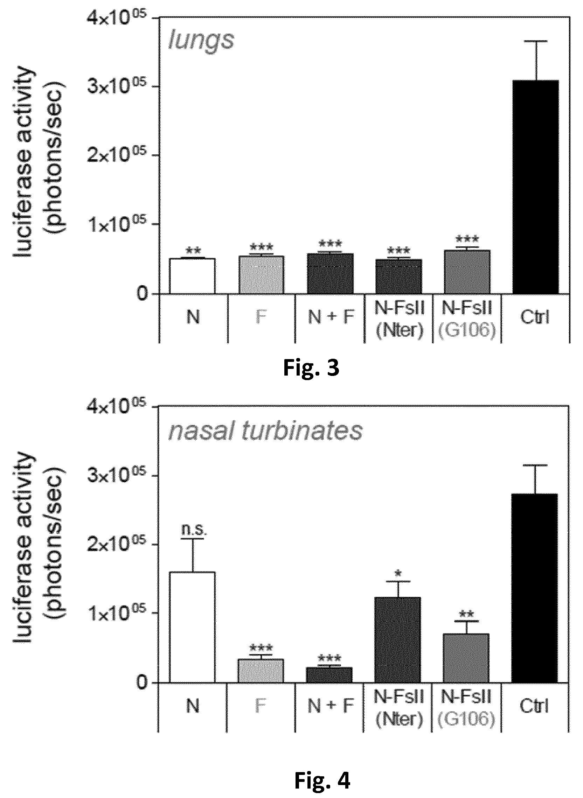

Figure 3: Evaluation of the protective efficacy of N-Fsll chimeric nanorings against RSV infection in mice in lungs. BALB/c mice were immunized as described in example 2. Two weeks after the boost immunization, mice were challenged with 8.8 X 10⁴ PFU's of RSV-Luciferase. Viral replication in lungs was evaluated at day 4 post-infection by measuring luciferase activity in living mice as described in example 3. Data are mean + SEM of individual values expressed in photons per second (n = 5-6 per experimental group). The significance observed between the control group (Ctrl) and each of the other group was evaluated according to the Student's T test (*, P<0.05; **, P<0.01; ***, P<0.001; n.s., non-significant).
Figure 4: Evaluation of the protective efficacy of N-Fsll chimeric nanorings against RSV infection in mice in nasal turbinates. BALB/c mice were immunized as described in example 2. Two weeks after the boost immunization, mice were challenged with 8.8 X 10⁴ PFU's of RSV-Luciferase. Nasal turbinates was evaluated at day 4 post-infection by measuring luciferase activity in living mice as described in example 3. Data are mean + SEM of individual values expressed in photons per second (n = 5-6 per experimental group). The significance observed between the control group (Ctrl) and each of the other group was evaluated according to the Student's T test (*, P<0.05; **, P<0.01; ***, P<0.001; n.s., non-significant).

### EXAMPLES

### Example 1: Design and characterization of N nanorings carrying F epitope site II (N-Fsll).

Two anchoring sites were investigated on N protein: N terminus (Nter) and a site on the loop of the β hairpin of the N terminal domain (position G106-K107).

N-Fsll (Nter) and N-Fsll (G106) fusion proteins were obtained by insertion of one Fsll epitope (SEQ ID NO: 6, i.e. STYMLTNSELLSLINDMPITNDQKKLMSNNVQIVRQQS) either at the N-terminus of N protein or between amino acid residues G106 and K107, respectively. For the construction of N-Fsll (Nter), the cDNA coding for Fsll epitope (Long strain) was amplified by PCR using DreamTaqTM DNA polymerase (Thermo Scientific, Waltham, Massachusetts) and cloned at Nhel and BamH1 restriction sites in pET-N plasmid. The plasmid generated was named pET-N-Fsll (Nter). For the construction of N-Fsll (G106), a Kpnl restriction site was created by site-directed mutagenesis at nucleotides 316-321 (T107K substitution) using the QuickChange® kit (Agilent Technologies, Santa Clara, California), in which the amplified cDNA coding for Fsll was inserted after the sequence coding for glycine 106 (G106). The plasmid generated was named pET-N-Fsll (G106). The entire sequences coding for N-Fsll (Nter) and N-Fsll (G106) were validated on both strands by DNA sequencing.

E.coli BL21 (DE3) bacteria were co-transformed with pGEX-PCT and pET-N-Fsll (Nter) or pET-N-Fsll (G106) and then grown at 37°C for 8 h in 1 L of Luria-Bertani (LB) medium containing 100 µg/mL of ampicillin and 50 µg/mL of kanamycin. The same volume of LB medium was then added, and protein expression was induced by adding 80 µg/mL isopropyl-β-D-thiogalactopyranoside (IPTG) to the medium. Bacteria were incubated for a further 15 h at 28°C and harvested by centrifugation. The purification of the recombinant N protein is permitted by its specific interaction with the C-terminal region of RSV P protein (PCT) (residues 161-241) fused to GST.

Protein complexes were purified from the bacterial pellets on glutathione-Sepharose 4B beads (GE Healthcare, Uppsala, Sweden). To isolate chimeric N nanorings, beads containing bound complexes were incubated with biotinylated thrombin (Merck Millipore, Darmstadt, Germany) for 16 h at 20°C. Thrombin was then removed using the cleavage capture kit according to the manufacturer's instructions (Merck Millipore, Darmstadt, Germany). Purified chimeric N nanorings were then concentrated on Vivaspin® centrifugal concentrator (Sartorius, Goettingen, Germany) and sterilely filtered. The protein sequence of the two purified N nanorings was additionally controlled by mass spectrometry (MALDI-TOF).

The two N-Fsll proteins [N-Fsll (Nter) and N-Fsll (G106)] were then analysed by SDS-PAGE. A single band migrating at the expected size was obtained for both N-Fsll constructs.

The capacity of N-Fsll (Nter) and N-Fsll (G106) to assemble as nanorings was assessed by Native Gel Electrophoresis (NGE) and Dynamic Light Scattering (DLS). NGE revealed two bands corresponding to nanorings made of 10 and 11 protomers as previously shown for epitope-free N protein. DLS plot showed one major peak for both N-Fsll (Nter) and N-Fsll (G106) indicative of homogeneous population of nanorings with a hydrodynamic diameter of 15 nm. Analysis of the protein content of N-Fsll nanorings was carried out by Western blot using anti-N polyclonal antibody or Fsll specific monoclonal antibodies (mAb11 and AbD Serotec), validating the presence of the F epitopes. Of note, a minor band migrating like N was observed for N-Fsll (Nter), as revealed by anti-N antibody but not by anti-F antibodies (pointed by an arrow on the top left panel). This might correspond to a partial degradation of N-Fsll (Nter) resulting in a loss of Fsll epitopes.

Finally, the effective presence of the Fsll epitopes on N nanorings was confirmed by indirect ELISA using N, N-Fsll (Nter) or N-Fsll (G106) as coating and pooled sera collected from mice immunized with postfusion F (F), Fsll-specific monoclonal antibody (AbD Serotec) (Fig. 1A) and palivizumab (Fig. 1 B). Interestingly, palivizumab presented a significantly increased affinity for N-Fsll (G106) as compared to N-Fsll (Nter) (Kd of 0.08 ± 0.02 nM and 0.53 ± 0.09 nM, respectively), suggesting a better recognition of Fsll epitope fused at G106 position (Fig. 1 B).

Overall, these results indicate that the fusion of Fsll to N protein at Nter or G106 positions allowed the formation of N-Fsll nanorings. Moreover, N-Fsll chimeric nanorings are efficiently recognized by anti-F antibodies.

### Example 2: N-Fsll nanorings induced anti-N and anti-F antibody responses in mice.

BALB/c mice were immunized twice intranasally with 2 µg of postfusion F (F), N nanorings associated with postfusion F (N+F), N-Fsll chimeric nanorings or N nanorings alone (N). All immunogens were adjuvanted with 5 % of MontanideTM gel (Seppic) and the negative control group (Ctrl) received adjuvant only.

Anti-N and anti-F antibody responses were evaluated in sera before and two weeks after the first and the second immunizations (Fig. 2). Groups of mice immunized with F or N+F presented high levels of F antibody titers (6.5 ± 0.2 log10 for IgG1 and 4.8 ± 0.3 log10 for IgG2a after two immunizations) or N and F antibody titers (6.3 ± 0.1 log10 for anti-N IgG1, 5.0 ± 0.1 log10 for anti-N IgG2a, 6.4 ± 0.1 log10 for anti-F IgG1 and 4.7 ± 0.5 log10 for anti-F IgG2a after two immunizations), respectively (Fig. 2A and 2B). Conversely, immunization with N-Fsll induced high level of anti-N antibody titers (N-Fsll (Nter): 6.4 ± 0.1 log10 for IgG1 and 5.3 ± 0.3 logs10 for IgG2a after two immunizations; N-Fsll (G106): 6.4 ± 0.1 log10 for IgG1 and 5.2 ± 0.2 log10 for IgG2a after two immunizations) and significant levels of anti-F antibody titers albeit at lower levels than after immunizations with F (P<0.001). Interestingly, the anti-F antibody response was significantly stronger in the group of mice immunized with N-Fsll (G106) (3.0 ± 0.8 log10 for IgG1 after the first immunization and 4.8 ± 0.2 log10 for IgG1 after the second immunization) than with N-Fsll (Nter) (1.5 ± 0.1 log10 for IgG1 after the first immunization and 2.9 ± 0.8 log10 for IgG1 after the second immunization) (P<0.01 after the first immunization and P<0.001 after the second immunization for IgG1 isotype). This suggests that the insertion of Fsll between residues G106 and K107 of N provided a better preservation of its antigenic conformation (Fig. 2B).

Broncho-alveolar lavage fluids (BALf) were collected from mice immunized as described above, two weeks after the second immunization. Anti-F Ig(H+L) and IgA were measured. Mice immunized intranasally with F and N+F displayed the highest titers of anti-F Ig(H+L) antibodies (4.7 ± 0.2 log10 and 4.6 ± 0.3 log10, respectively) and IgA antibodies (2.9 ± 0.2 log10 and 2.7 ± 0.4 log10, respectively). Immunization with N-Fsll chimeric nanorings gave rise to significant levels of anti-F Ig(H+L) antibody titers (N-Fsll (Nter): 1.7 ± 0.6 log10; N-Fsll (G106): 2.8 ± 0.6 log10). Interestingly, in accordance with the antibody responses measured in serum, anti-F antibody titers were significantly higher in N-Fsll (G106) vaccinated group as compared to N-Fsll (Nter) vaccinated group (P<0.01 for Ig(H+L) antibody titers).

Overall, these results indicate that N-Fsll were potently immunogenic in mice and induced both systemic and local anti-N and anti-F antibody responses. Moreover, N-Fsll (G106) presented a significant advantage for the induction of anti-F antibody response as compared to N-Fsll (Nter).

### Example 3: Immunization with N-Fsll nanorings protected mice against RSV challenge.

The inventors used a luciferase-expressing recombinant RSV (RSV-Luciferase) for viral challenge. This recombinant virus allowed monitoring of virus replication independently and simultaneously in the lower and upper airways of living mice, using luminescence detection. First, they evaluated the kinetics of virus replication in lungs and nasal turbinates of non-vaccinated mice. The peak viral load was identified at day 4 post-infection at both sites of replication. Importantly, they demonstrated that vaccination with F afforded a complete viral protection in both lower and upper airways, from day 3 up to day 6 post-infection. Thus they investigated whether N-Fsll could provide similar levels of viral protection.

Mice immunized as described in example 2 were challenged with RSV-Luciferase two weeks after the boost immunization. Viral replication was monitored in lungs and in nasal turbinates at the peak of replication (day 4 post-infection) via luminescence detection in living animals. All groups of vaccinated mice (N, F, N+F and N-Fsll (G106 or Nter)) were totally protected against viral replication in lungs (Fig. 3). This result was confirmed by the measure of luminescence in lung homogenates collected at autopsy at day 5 post-infection (data not shown).

However, when measuring the luminescence in the nasal turbinates, the inventors recorded significant differences in the viral replication between vaccinated groups. For this nasal site, the best protection was afforded by F and N+F immunizations whereas N immunization failed to reduce significantly viral replication (P=0.1) (Fig. 4). Interestingly, N-Fsll (Nter) and especially N-Fsll (G106) immunizations significantly reduced viral replication in upper airways as compared to control group (P=0.01 and P=0.001 respectively) (Fig. 4). In agreement with the absence of RSV replication in lungs, all vaccinated mice were protected against weight loss and clinical symptoms after challenge, as opposed to the significant weight loss (9 % of initial weight at day 3 post-infection, P<0.01) and clinical symptoms (P<0.001) recorded in the control group.

These results suggested that the clinical symptoms observed in control mice were correlated to the replication rate of RSV virus in lungs rather than in nasal turbinates.

Mice were sacrificed at day 5 post-infection and lung leukocytes were enumerated from bronchoalveolar lavages (BAL). All vaccinated mice presented a significant but moderate increase of eosinophils as compared to the control group. Of note, all vaccinated groups displayed a slight increase of lymphocytes as compared to the control group, which was significant for N-Fsll vaccinated group (P=0.02).

Overall, these results indicate that immunization of mice with N-Fsll gave rise to significant virological and clinical protection against RSV challenge, associated to a mild inflammatory reaction.

## Claims

1. A fusion protein comprising a N protein from a virus of the *Pneumovirinae* subfamily and a peptide of interest, said peptide of interest being inserted in frame at the loop of the β hairpin of the N terminal domain of the N protein in the region corresponding to residues 103-109 of human RSV N protein represented by SEQ ID NO: 1.

2. The fusion protein according to claim 1, wherein the virus of the *Pneumovirinae* subfamily is human or bovine respiratory syncytial virus.

3. The fusion protein according to any one of claims 1 to 2, wherein the N protein is a native protein or a N protein modified in the region defined by the last 25 C-terminal amino acids, said modified N protein retaining the ability to interact with the P protein.

4. The fusion protein according to any one of claims 1 to 3, wherein the peptide of interest is an antigen, preferably an epitope, more preferably a conformational epitope.

5. The fusion protein according to claim 4, wherein the peptide of interest is an epitope derived from the F protein from a virus of the *Pneumovirinae* subfamily, preferably from RSV.

6. A complex comprising a fusion protein according to any one of claims 1 to 5 and a P protein, wherein the N and the P protein are from the same virus of the *Pneumovirinae* subfamily.

7. A nucleic acid coding for a fusion protein as defined in any one of claims 1 to 5.

8. An expression vector comprising a nucleic acid as defined in claim 7 and preferably further comprising a nucleic acid coding for a P protein from the same virus of the *Pneumovirinae* subfamily as the N protein of the fusion protein.

9. A host cell containing:
(i) an expression vector as defined in claim 8, or
(ii) a pair of expression vectors comprising a first expression vector as defined in claim 8 and a second expression vector comprising a nucleic acid coding for a P protein from the same virus of the *Pneumovirinae* subfamily as the N protein of the fusion protein encoded by the first expression vector.

10. A method for producing a fusion protein according to any one of claims 1 to 5, said method comprising the culture of a host cell as claimed in claim 9 under conditions permitting the expression of fusion protein, and optionally the purification of the expressed fusion protein.

11. A method for preparing fusion protein / P protein complexes according to claim 6, said method comprising the steps consisting in:
a) coexpressing a fusion protein as defined in any one of claims 1 to 5, with a P protein from the same virus of the *Pneumovirinae* subfamily as the N protein of the fusion protein;
b) collecting the so formed fusion protein /P protein complexes.

12. A method for preparing a fusion protein according to any one of claims 1 to 5, said method comprising the separation of the protein fusion starting from the complex according to claim 6.

13. A pharmaceutical composition comprising a fusion protein as defined in any one of claims 1 to 5 and/or a complex as defined in claim 6, and a pharmaceutically acceptable carrier, in which the pharmaceutical composition is preferably an immunogenic or vaccinal composition.

14. The fusion protein according to any one of claims 1 to 5 or the complex as defined in claim 6, for use as a vaccine.

15. The fusion protein or the complex for its use according to claim 14, wherein the peptide of interest of the fusion protein is an epitope derived from the RSV F protein, for use for preventing bronchiolitis or pneumonia.


SEQUENCE LISTING

<110>  Institut National de la Recherche Agronomique

<120>  Fusion protein comprising the N protein of a virus of the
       Pneumovirinae subfamily

<130>  BET15P3219

<160>  9

<170>  PatentIn version 3.5

<210>  1
<211>  391
<212>  PRT
<213>  Human respiratory syncytial virus

<400>  1

Met Ala Leu Ser Lys Val Lys Leu Asn Asp Thr Leu Asn Lys Asp Gln
1               5                   10                  15

Leu Leu Ser Ser Ser Lys Tyr Thr Ile Gln Arg Ser Thr Gly Asp Ser
            20                  25                  30

Ile Asp Thr Pro Asn Tyr Asp Val Gln Lys His Ile Asn Lys Leu Cys
        35                  40                  45

Gly Met Leu Leu Ile Thr Glu Asp Ala Asn His Lys Phe Thr Gly Leu
    50                  55                  60

Ile Gly Met Leu Tyr Ala Met Ser Arg Leu Gly Arg Glu Asp Thr Ile
65                  70                  75                  80

Lys Ile Leu Arg Asp Ala Gly Tyr His Val Lys Ala Asn Gly Val Asp
                85                  90                  95

Val Thr Thr His Arg Gln Asp Ile Asn Gly Lys Glu Met Lys Phe Glu
            100                 105                 110

Val Leu Thr Leu Ser Ser Leu Thr Thr Glu Ile Gln Ile Asn Ile Glu
        115                 120                 125

Ile Glu Ser Arg Lys Ser Tyr Lys Lys Met Leu Lys Glu Met Gly Glu
    130                 135                 140

Val Ala Pro Glu Tyr Arg His Asp Ser Pro Asp Cys Gly Met Ile Ile
145                 150                 155                 160

Leu Cys Ile Ala Ala Leu Val Ile Thr Lys Leu Ala Ala Gly Asp Arg
                165                 170                 175

Ser Gly Leu Thr Ala Val Ile Arg Arg Ala Asn Asn Val Leu Lys Asn
            180                 185                 190

Glu Met Lys Arg Tyr Lys Gly Leu Leu Pro Lys Asp Ile Ala Asn Ser
        195                 200                 205

Phe Tyr Glu Val Phe Glu Lys Tyr Pro His Phe Ile Asp Val Phe Val
    210                 215                 220

His Phe Gly Ile Ala Gln Ser Ser Thr Arg Gly Gly Ser Arg Val Glu
225                 230                 235                 240

Gly Ile Phe Ala Gly Leu Phe Met Asn Ala Tyr Gly Ala Gly Gln Val
                245                 250                 255

Met Leu Arg Trp Gly Val Leu Ala Lys Ser Val Lys Asn Ile Met Leu
            260                 265                 270

Gly His Ala Ser Val Gln Ala Glu Met Glu Gln Val Val Glu Val Tyr
        275                 280                 285

Glu Tyr Ala Gln Lys Leu Gly Gly Glu Ala Gly Phe Tyr His Ile Leu
    290                 295                 300

Asn Asn Pro Lys Ala Ser Leu Leu Ser Leu Thr Gln Phe Pro His Phe
305                 310                 315                 320

Ser Ser Val Val Leu Gly Asn Ala Ala Gly Leu Gly Ile Met Gly Glu
                325                 330                 335

Tyr Arg Gly Thr Pro Arg Asn Gln Asp Leu Tyr Asp Ala Ala Lys Ala
            340                 345                 350

Tyr Ala Glu Gln Leu Lys Glu Asn Gly Val Ile Asn Tyr Ser Val Leu
        355                 360                 365

Asp Leu Thr Ala Glu Glu Leu Glu Ala Ile Lys His Gln Leu Asn Pro
    370                 375                 380

Lys Asp Asn Asp Val Glu Leu
385                 390

<210>  2
<211>  391
<212>  PRT
<213>  Bovine respiratory syncytial virus

<400>  2

Met Ala Leu Ser Lys Val Lys Leu Asn Asp Thr Phe Asn Lys Asp Gln
1               5                   10                  15

Leu Leu Ser Thr Ser Lys Tyr Thr Ile Gln Arg Ser Thr Gly Asp Asn
            20                  25                  30

Ile Asp Ile Pro Asn Tyr Asp Val Gln Lys His Leu Asn Lys Leu Cys
        35                  40                  45

Gly Met Leu Leu Ile Thr Glu Asp Ala Asn His Lys Phe Thr Gly Leu
    50                  55                  60

Ile Gly Met Leu Tyr Ala Met Ser Arg Leu Gly Arg Glu Asp Thr Leu
65                  70                  75                  80

Lys Ile Leu Lys Asp Ala Gly Tyr Gln Val Arg Ala Asn Gly Val Asp
                85                  90                  95

Val Ile Thr His Arg Gln Asp Val Asn Gly Lys Glu Met Lys Phe Glu
            100                 105                 110

Val Leu Thr Leu Val Ser Leu Thr Ser Glu Val Gln Gly Asn Ile Glu
        115                 120                 125

Ile Glu Ser Arg Lys Ser Tyr Lys Lys Met Leu Lys Glu Met Gly Glu
    130                 135                 140

Val Ala Pro Glu Tyr Arg His Asp Phe Pro Asp Cys Gly Met Ile Val
145                 150                 155                 160

Leu Cys Val Ala Ala Leu Val Ile Thr Lys Leu Ala Ala Gly Asp Arg
                165                 170                 175

Ser Gly Leu Thr Ala Val Ile Arg Arg Ala Asn Asn Val Leu Arg Asn
            180                 185                 190

Glu Met Lys Arg Tyr Lys Gly Leu Ile Pro Lys Asp Ile Ala Asn Ser
        195                 200                 205

Phe Tyr Glu Val Phe Glu Lys Tyr Pro His Tyr Ile Asp Val Phe Val
    210                 215                 220

His Phe Gly Ile Ala Gln Ser Ser Thr Arg Gly Gly Ser Arg Val Glu
225                 230                 235                 240

Gly Ile Phe Ala Gly Leu Phe Met Asn Ala Tyr Gly Ala Gly Gln Val
                245                 250                 255

Met Leu Arg Trp Gly Val Leu Ala Lys Ser Val Lys Asn Ile Met Leu
            260                 265                 270

Gly His Ala Ser Val Gln Ala Glu Met Glu Gln Val Val Glu Val Tyr
        275                 280                 285

Glu Tyr Ala Gln Lys Leu Gly Gly Glu Ala Gly Phe Tyr His Ile Leu
    290                 295                 300

Asn Asn Pro Lys Ala Ser Leu Leu Ser Leu Thr Gln Phe Pro Asn Phe
305                 310                 315                 320

Ser Ser Val Val Leu Gly Asn Ala Ala Gly Leu Gly Ile Met Gly Glu
                325                 330                 335

Tyr Arg Gly Thr Pro Arg Asn Gln Asp Leu Tyr Asp Ala Ala Lys Ala
            340                 345                 350

Tyr Ala Glu Gln Leu Lys Glu Asn Gly Val Ile Asn Tyr Ser Val Leu
        355                 360                 365

Asp Leu Thr Thr Glu Glu Leu Glu Ala Ile Lys Asn Gln Leu Asn Pro
    370                 375                 380

Lys Asp Asn Asp Val Glu Leu
385                 390

<210> 3
<211> 394
<212> PRT
<213> Human Metapneumovirus

<400> 3

Met Ser Leu Gln Gly Ile His Leu Ser Asp Leu Ser Tyr Lys His Ala
1               5                   10                  15

Ile Leu Lys Glu Ser Gln Tyr Thr Ile Lys Arg Asp Val Gly Thr Thr
            20                  25                  30

Thr Ala Val Thr Pro Ser Ser Leu Gln Gln Glu Ile Thr Leu Leu Cys
        35                  40                  45

Gly Glu Ile Leu Tyr Ala Lys His Ala Asp Tyr Lys Tyr Ala Ala Glu
    50                  55                  60

Ile Gly Ile Gln Tyr Ile Ser Thr Ala Leu Gly Ser Glu Arg Val Gln
65                  70                  75                  80

Gln Ile Leu Arg Asn Ser Gly Ser Glu Val Gln Val Val Leu Thr Arg
                85                  90                  95

Thr Tyr Ser Leu Gly Lys Ile Lys Asn Asn Lys Gly Glu Asp Leu Gln
            100                 105                 110

Met Leu Asp Ile His Gly Val Glu Lys Ser Trp Val Glu Glu Ile Asp
        115                 120                 125

Lys Glu Ala Arg Lys Thr Met Ala Thr Leu Leu Lys Glu Ser Ser Gly
    130                 135                 140

Asn Ile Pro Gln Asn Gln Arg Pro Ser Ala Pro Asp Thr Pro Ile Ile
145                 150                 155                 160

Leu Leu Cys Val Gly Ala Leu Ile Phe Thr Lys Leu Ala Ser Thr Ile
                165                 170                 175

Glu Val Gly Leu Glu Thr Thr Val Arg Arg Ala Asn Arg Val Leu Ser
            180                 185                 190

Asp Ala Leu Lys Arg Tyr Pro Arg Met Asp Ile Pro Lys Ile Ala Arg
        195                 200                 205

Ser Phe Tyr Asp Leu Phe Glu Gln Lys Val Tyr His Arg Ser Leu Phe
    210                 215                 220

Ile Glu Tyr Gly Lys Ala Leu Gly Ser Ser Ser Thr Gly Ser Lys Ala
225                 230                 235                 240

Glu Ser Leu Phe Val Asn Ile Phe Met Gln Ala Tyr Gly Ala Gly Gln
                245                 250                 255

Thr Met Leu Arg Trp Gly Val Ile Ala Arg Ser Ser Asn Asn Ile Met
            260                 265                 270

Leu Gly His Val Ser Val Gln Ala Glu Leu Lys Gln Val Thr Glu Val
        275                 280                 285

Tyr Asp Leu Val Arg Glu Met Gly Pro Glu Ser Gly Leu Leu His Leu
    290                 295                 300

Arg Gln Ser Pro Lys Ala Gly Leu Leu Ser Leu Ala Asn Cys Pro Asn
305                 310                 315                 320

Phe Ala Ser Val Val Leu Gly Asn Ala Ser Gly Leu Gly Ile Ile Gly

325                 330                 335

Met Tyr Arg Gly Arg Val Pro Asn Thr Glu Leu Phe Ser Ala Ala Glu
            340                 345                 350

Ser Tyr Ala Lys Ser Leu Lys Glu Ser Asn Lys Ile Asn Phe Ser Ser
        355                 360                 365

Leu Gly Leu Thr Asp Glu Glu Lys Glu Ala Ala Glu His Phe Leu Asn
    370                 375                 380

Val Ser Asp Asp Ser Gln Asn Asp Tyr Glu
385                 390

<210>  4
<211>  7
<212>  PRT
<213>  Human respiratory syncytial virus

<400>  4

Asp Ile Asn Gly Lys Glu Met
1               5

<210>  5
<211>  21
<212>  PRT
<213>  Human respiratory syncytial virus

<400>  5

Ser Glu Leu Leu Ser Leu Ile Asn Asp Met Pro Ile Thr Asn Asp Gln
1               5                   10                  15

Lys Lys Leu Met Ser
            20

<210>  6
<211>  38
<212>  PRT
<213>  Human respiratory syncytial virus

<400>  6

Ser Thr Tyr Met Leu Thr Asn Ser Glu Leu Leu Ser Leu Ile Asn Asp
1               5                   10                  15

Met Pro Ile Thr Asn Asp Gln Lys Lys Leu Met Ser Asn Asn Val Gln
            20                  25                  30

Ile Val Arg Gln Gln Ser
        35

<210>  7
<211>  241
<212>  PRT
<213>  Human respiratory syncytial virus

<400>  7

Met Glu Lys Phe Ala Pro Glu Phe His Gly Glu Asp Ala Asn Asn Arg
1               5                   10                  15

Ala Thr Lys Phe Leu Glu Ser Ile Lys Gly Lys Phe Thr Ser Pro Lys
            20                  25                  30

Asp Pro Lys Lys Lys Asp Ser Ile Ile Ser Val Asn Ser Thr Asp Ile
        35                  40                  45

Glu Val Thr Lys Glu Ser Pro Ile Thr Ser Asn Ser Thr Ile Ile Asn
    50                  55                  60

Pro Thr Asn Glu Thr Asp Asp Asn Ala Gly Asn Lys Pro Asn Tyr Gln
65                  70                  75                  80

Arg Lys Pro Leu Val Ser Phe Lys Glu Asp Pro Ile Pro Ser Asp Asn
                85                  90                  95

Pro Phe Ser Lys Leu Tyr Lys Glu Thr Ile Glu Thr Phe Asp Asn Asn
            100                 105                 110

Glu Glu Glu Ser Ser Tyr Ser Tyr Glu Glu Ile Asn Asp Gln Thr Asn
        115                 120                 125

Asp Asn Ile Thr Ala Arg Leu Asp Arg Ile Asp Glu Lys Leu Ser Glu
    130                 135                 140

Ile Leu Gly Met Leu His Thr Leu Val Val Ala Ser Ala Gly Pro Thr
145                 150                 155                 160

Ser Ala Arg Asp Gly Ile Arg Asp Ala Met Val Gly Leu Arg Glu Glu
                165                 170                 175

Met Ile Glu Lys Ile Arg Thr Glu Ala Leu Met Thr Asn Asp Arg Leu
            180                 185                 190

Glu Ala Met Ala Arg Leu Arg Asn Glu Glu Ser Glu Lys Met Ala Lys
        195                 200                 205

Asp Thr Ser Asp Glu Val Ser Leu Asn Pro Thr Ser Glu Lys Leu Asn
    210                 215                 220

Asn Leu Leu Glu Gly Asn Asp Ser Asp Asn Asp Leu Ser Leu Glu Asp
225                 230                 235                 240

Phe

<210>  8
<211>  241
<212>  PRT
<213>  Bovine respiratory syncytial virus

<400>  8

Met Glu Lys Phe Ala Pro Glu Phe His Gly Glu Asp Ala Asn Thr Lys
1               5                   10                  15

Ala Thr Lys Phe Leu Glu Ser Leu Lys Gly Lys Phe Thr Ser Ser Lys
            20                  25                  30

Asp Ser Arg Lys Lys Asp Ser Ile Ile Ser Val Asn Ser Ile Asp Ile
        35                  40                  45

Glu Leu Pro Lys Glu Ser Pro Ile Thr Ser Thr Asn His Asn Ile Asn
    50                  55                  60

Gln Pro Ser Glu Ile Asn Asp Thr Ile Ala Ala Asn Gln Val His Ile
65                  70                  75                  80

Arg Lys Pro Leu Val Ser Phe Lys Glu Glu Leu Pro Ser Ser Glu Asn
                85                  90                  95

Pro Phe Thr Lys Leu Tyr Lys Glu Thr Ile Glu Thr Phe Asp Asn Asn
            100                 105                 110

Glu Glu Glu Ser Ser Tyr Ser Tyr Asp Glu Ile Asn Asp Gln Thr Asn
        115                 120                 125

Asp Asn Ile Thr Ala Arg Leu Asp Arg Ile Asp Glu Lys Leu Ser Glu
    130                 135                 140

Ile Ile Gly Met Leu His Thr Leu Val Val Ala Ser Ala Gly Pro Thr
145                 150                 155                 160

Ala Ala Arg Asp Gly Ile Arg Asp Ala Met Val Gly Leu Arg Glu Glu
                165                 170                 175

Met Ile Glu Lys Ile Arg Ser Glu Ala Leu Met Thr Asn Asp Arg Leu
            180                 185                 190

Glu Ala Met Ala Arg Leu Arg Asp Glu Glu Ser Glu Lys Met Thr Lys
        195                 200                 205

Asp Thr Ser Asp Glu Val Lys Leu Thr Pro Thr Ser Glu Lys Leu Asn
    210                 215                 220

Met Val Leu Glu Asp Glu Ser Ser Asp Asn Asp Leu Ser Leu Glu Asp
225                 230                 235                 240

Phe

<210>  9
<211>  294
<212>  PRT
<213>  Human Metapneumovirus

<400>  9

Met Ser Phe Pro Glu Gly Lys Asp Ile Leu Phe Met Gly Asn Glu Ala
1               5                   10                  15

Ala Lys Leu Ala Glu Ala Phe Gln Lys Ser Leu Arg Lys Pro Gly His
            20                  25                  30

Lys Arg Ser Gln Ser Ile Ile Gly Glu Lys Val Asn Thr Val Ser Glu
        35                  40                  45

Thr Leu Glu Leu Pro Thr Ile Ser Arg Pro Ala Lys Pro Thr Ile Pro
    50                  55                  60

Ser Glu Pro Lys Leu Ala Trp Thr Asp Lys Gly Gly Ala Thr Lys Thr
65                  70                  75                  80

Glu Ile Lys Gln Ala Ile Lys Val Met Asp Pro Ile Glu Glu Glu Glu
                85                  90                  95

Ser Thr Glu Lys Lys Val Leu Pro Ser Ser Asp Gly Lys Thr Pro Ala
            100                 105                 110

Glu Lys Lys Leu Lys Pro Ser Thr Asn Thr Lys Lys Lys Val Ser Phe
        115                 120                 125

Thr Pro Asn Glu Pro Gly Lys Tyr Thr Lys Leu Glu Lys Asp Ala Leu
    130                 135                 140

Asp Leu Leu Ser Asp Asn Glu Glu Glu Asp Ala Glu Ser Ser Ile Leu
145                 150                 155                 160

Thr Phe Glu Glu Arg Asp Thr Ser Ser Leu Ser Ile Glu Ala Arg Leu
                165                 170                 175

Glu Ser Ile Glu Glu Lys Leu Ser Met Ile Leu Gly Leu Leu Arg Thr
            180                 185                 190

Leu Asn Ile Ala Thr Ala Gly Pro Thr Ala Ala Arg Asp Gly Ile Arg
        195                 200                 205

Asp Ala Met Ile Gly Val Arg Glu Glu Leu Ile Ala Asp Ile Ile Lys
    210                 215                 220

Glu Ala Lys Gly Lys Ala Ala Glu Met Met Glu Glu Glu Met Ser Gln
225                 230                 235                 240

Arg Ser Lys Ile Gly Asn Gly Ser Val Lys Leu Thr Glu Lys Ala Lys
                245                 250                 255

Glu Leu Asn Lys Ile Val Glu Asp Glu Ser Thr Ser Gly Glu Ser Glu
            260                 265                 270

Glu Glu Glu Glu Pro Lys Asp Thr Gln Asp Asn Ser Gln Glu Asp Asp
        275                 280                 285

Ile Tyr Gln Leu Ile Met
    290